# EUROPÄISCHE PATENTSCHRIFT

(11) **EP 3 541 330 B1**
(45) Veröffentlichungstag und Bekanntmachung des Hinweises auf die Patenterteilung: **17.03.2021**
(21) Anmeldenummer: 17840549.4
(22) Anmeldetag: 14.11.2017
(51) Int. Cl.: A61F 5/02, B25J 9/00

(54) **SYSTEM UND VERFAHREN ZUR REDUKTION VON AUF EINE WIRBELSÄULE WIRKENDEN KRÄFTEN**
SYSTEM AND METHOD TO REDUCE FORCES ON THE SPINE
SYSTEME ET METHODE POUR REDUIRE DES FORCES SUR LA COLONNE VERTEBRALE

(30) Priorität: 18.11.2016 DE 102016122282
(43) Veröffentlichungstag der Anmeldung: 25.09.2019
(73) Patentinhaber: exoIQ GmbH, 21109 Hamburg (DE)
(72) Erfinder: WEIDNER, Robert, 22043 Hamburg (DE); MEYER, Tobias, 22043 Hamburg (DE); WULFSBERGER, Jens Peter, 22043 Hamburg (DE)
(74) Vertreter: Koplin, Moritz
(86) Internationale Anmeldenummer: PCT/DE2017/100966
(87) Internationale Veröffentlichungsnummer: WO 2018/091037

(56) Entgegenhaltungen:
- WO-A1-2005/105004
- WO-A1-2015/078981
- WO-A1-2016/015070
- DE-A1-102011 076 843

## Beschreibung

### Gebiet der Technik

Die vorliegende Erfindung betrifft ein anziehbares, modulares, (exoskelettales) System, welches bspw. auf dem Rücken eines Menschen parallel zur Wirbelsäule getragen werden kann, um die Belastung der Wirbelsäule bzw. der Wirbelsäulenmuskulatur bei ergonomisch ungünstigen Tätigkeiten wie bspw. Lasthandhabungsprozessen zu reduzieren oder Bewegungen von Personen mit Muskel-Skelett-Erkrankungen zu unterstützen oder auf einen bestimmten Bereich zu begrenzen. Insbesondere dient das System der Kraftumleitung zur Entlastung der Wirbelsäule bzw. der Wirbelsäulenmuskulatur.

### Stand der Technik

Aus dem Stand der Technik sind zahlreiche technische Systeme bekannt, die menschliche Bewegungen unterstützen, um z. B. die Qualität bei der Ausführung manueller Arbeitsschritte, die Arbeitsplatz-Ergonomie oder die Mobilität von Personen mit Muskel-Skelett-Erkrankungen zu verbessern. Die Systeme adressieren dabei den ganzen Körper oder einzelne Körperbereiche, wie bspw. die unteren Extremitäten, die oberen Extremitäten oder den Rücken.

Beispiele für aktuierte (aktive) Systeme sind der Hybrid Assistive Limb (HAL), der ReWalk, das Exoskelett der Firma Boston Dynamics oder das Unterstützungssystem Lucy, welches zur Unterstützung von Tätigkeiten in und über Kopfhöhe einsetzbar ist. Alle diese Systeme haben gemeinsam, dass sie die Bewegung der unteren und/oder oberen Extremitäten unterstützen. Ein Beispiel für nicht-aktuierte (passive) Systeme sind Orthesen, wie bspw. Rückenorthesen zur Stabilisierung der Wirbelsäule bei Wirbelverletzungen. Orthesen nutzen oftmals steife oder halbsteife Rückenstrukturen, die die Bewegungsfreiheit gezielt begrenzen. Ein weiteres Beispiel für nicht-aktuierte (passive) Systeme ist aus der WO 2016/015070 A1 bekannt. Weiterer Stand der Technik findet sich in der DE 10 2011 076843 A1, der WO 2005/10500 A1 und der WO 2015/078981 A1.

Obwohl somit aktive und passive Systeme für vielfältige Einsatzszenarien bekannt sind, verbleibt Raum für Verbesserungen in Hinblick auf das Ermöglichen eines möglichst natürlichen Bewegungsablaufs.

### Zusammenfassung der Erfindung

Es ist die Aufgabe der vorliegenden Erfindung, die aus dem Stand der Technik bekannten Systeme zu verbessern.

Diese Aufgabe wird durch ein erfindungsgemäßes System , wie in den Ansprüchen definiert, gelöst. Das erfindungsgemäße System weist eine Vielzahl von aneinander anreihbaren Exoskelett-Elementen auf, wobei zumindest zwei der aneinander anreihbaren Exoskelett-Elemente eingerichtet sind, während der Verwendung des Systems an den Körper des Menschen angebunden zu sein, und ein erstes Exoskelett-Element der aneinander anreihbaren Exoskelett-Elemente eine Führung umfasst, welche eingerichtet ist, ein zweites Exoskelett-Element der aneinander anreihbaren Exoskelett-Elemente entlang einer gekrümmten Trajektorie relativ zum ersten Exoskelett-Element zu führen und beim Führen des zweiten Exoskelett-Elements entlang der gekrümmten Trajektorie relativ zum ersten Exoskelett-Element eine überlagerte translatorische und rotatorische Relativbewegung zwischen dem ersten Exoskelett-Element und dem zweiten Exoskelett-Element zu erzwingen, umfassend ferner eine Aktuator-Einheit, wobei die Aktuator-Einheit zum steuerbaren Verschieben des zweiten Exoskelett-Elements relativ zum ersten Exoskelett-Element eingerichtet ist.

Die aneinander anreihbaren Exoskelett-Elemente können somit relativ zueinander verschoben werden, wobei sich während der Verschiebung der Abstand und die Ausrichtung der Exoskelett-Elemente zueinander (in vorgegebenen Grenzen) ändern, d. h. unterschiedliche Abstände mit unterschiedlichen Ausrichtungen korrelieren. Bspw. kann bei einem ersten Abstand eine erste Ausrichtung und bei einem zweiten Abstand eine zweite Ausrichtung erzwungen oder zumindest ermöglicht werden, während bei dem ersten Abstand die zweite Ausrichtung und beim zweiten Abstand die erste Ausrichtung verhindert wird.

In diesem Zusammenhang ist unter dem Begriff "aneinander anreihbar", wie er in der Beschreibung und den Ansprüchen verwendet wird, insbesondere das Eingerichtet sein, zum Eingehen einer (lösbaren) seriellen Verbindung oder Verkettung zu verstehen. Ferner ist unter dem Begriff "Exoskelett-Element", wie er in der Beschreibung und den Ansprüchen verwendet wird, insbesondere ein Bauteil zu verstehen, welches bei der Verwendung des Systems dazu beiträgt eine Stütz-, Halte- oder Kraftübertragungsfunktion hinsichtlich des menschlichen Körpers zu realisieren.

Des Weiteren ist unter dem Begriff "angebunden", wie er in der Beschreibung und den Ansprüchen verwendet wird, insbesondere ein Kontaktieren zu verstehen, bei dem auf die Wirbelsäule wirkende Kräfte umgeleitet werden können, bspw. indem das System im Schulter-/Brustbereich und im Hüft-/Schambeinbereich anliegt bzw. diesen (teilweise) umschließt oder umgreift. Zudem ist unter dem Begriff "Trajektorie", wie er in der Beschreibung und den Ansprüchen verwendet wird, insbesondere ein zusammenhängender Pfad zu verstehen.

Das System umfasst ferner eine Aktuator-Einheit, wobei die Aktuator-Einheit zum steuerbaren Verschieben des zweiten Exoskelett-Elements relativ zum ersten Exoskelett-Element eingerichtet ist.

Vorzugsweise schneidet beim Führen des zweiten Exoskelett-Elements entlang der gekrümmten Trajektorie relativ zum ersten Exoskelett-Element die Gangpolbahn des zweiten Exoskelett-Elements das erste und das zweite Exoskelett-Element nicht.

Vorzugsweise liegt beim Führen des zweiten Exoskelett-Elements entlang der gekrümmten Trajektorie relativ zum ersten Exoskelett-Element die Gangpolbahn des zweiten Exoskelett-Elements relativ zum ersten und zweiten Exoskelett-Element wirbelsäulenseitig versetzt.

Vorzugsweise folgt beim Führen des zweiten Exoskelett-Elements entlang der gekrümmten Trajektorie relativ zum ersten Exoskelett-Element der Momentanpol des zweiten Exoskelett-Elements dem Momentanpol eines Wirbels oder einer Gruppe von Wirbeln der Wirbelsäule oder stimmt mit dem Momentanpol des Wirbels bzw. der Gruppe von Wirbeln überein.

Vorzugsweise ist ein erstes Führungselement des zweiten Exoskelett-Elements in der Führung gleitend lagerbar, wobei das zweite Exoskelett-Element bei einer Verschiebung des ersten Führungselements in der Führung entlang der gekrümmten Trajektorie relativ zum ersten Exoskelett-Element geführt wird.

Durch eine entsprechende Ausgestaltung der Trajektorie kann dadurch erreicht werden, dass eine Abweichung zwischen der Momentanpolbewegung der Wirbel der Wirbelsäule des Menschen und der Momentanpolbewegung entsprechender Exoskelett-Elemente (im Wesentlichen) auf null reduziert wird und somit beim Beugen und Strecken der Wirbelsäule keinerlei oder nur verschwindend geringe Relativbewegungen zwischen den Anbindungspunkten des Systems und dem menschlichen Körper auftreten.

Vorzugsweise weist das zweite Exoskelett-Element ein Grundelement auf und das erste Führungselement des zweiten Exoskelett-Elements ist relativ zum Grundelement drehbar gelagert, wobei eine Drehung des ersten Führungselements des zweiten Exoskelett-Elements relativ zum Grundelement es ermöglicht, das zweite Exoskelett-Element entlang einer zweiten gekrümmten Trajektorie relativ zum ersten Exoskelett-Element zu führen.

Dadurch wird es ermöglicht, neben einem Beugen und Strecken der Wirbelsäule auch komplexere Bewegungen, bspw. ein seitliches Neigen oder ein Drehen um die Hochachse der Wirbelsäule, zu ermöglichen.

Vorzugsweise ist ein zweites Führungselement des zweiten Exoskelett-Elements in der Führung gleitend lagerbar, wobei das erste Führungselement und das zweite Führungselement voneinander entlang einer Geraden beabstandet sind.

Dadurch kann die Stabilität der Bewegung erhöht und der Verschleiß des Systems reduziert werden.

Vorzugsweise liegt die Gerade während der Verwendung des Systems in einer durch einen Abschnitt der gekrümmten Trajektorie aufgespannten Ebene.

Durch das Führen der Führungselemente mittels zweier separater Führungselemente, die entlang unterschiedlicher Führungspfade oder unterschiedlicher Abschnitte eines Führungspfads geführt werden, können neben bspw. kreisrunden Trajektorien beliebig komplexe Korrelationen zwischen Abstand und Ausrichtung erreicht werden.

Vorzugsweise ist ein Abstand des ersten Führungselements zum zweiten Führungselement oder zum Schwerpunkt des zweiten Exoskelett-Elements stufenlos oder in Stufen einstellbar, wobei unterschiedliche Abstände unterschiedlich gekrümmte Trajektorien bewirken.

Durch die Einstellbarkeit der Trajektorienkrümmung kann eine Abweichung zwischen der Momentanpolbewegung der Wirbel der Wirbelsäule des Menschen und der Momentanpolbewegung entsprechender Exoskelett-Elemente (im Wesentlichen) auf null reduziert werden, ohne maßgefertigte Exoskelett-Elemente verwenden zu müssen.

Vorzugsweise ist die Führung stufenlos oder in Stufen einstellbar, wobei unterschiedliche Einstellungen unterschiedlich gekrümmte Trajektorien bewirken.

Durch die Einstellbarkeit der Trajektorienkrümmung kann, wie bereits ausgeführt, eine Abweichung zwischen einer Momentanpolbewegung der Wirbel der Wirbelsäule des Menschen und einer Momentanpolbewegung entsprechender Exoskelett-Elemente (im Wesentlichen) auf null reduziert werden, ohne maßgefertigte Exoskelett-Elemente verwenden zu müssen.

Vorzugsweise sind das erste Exoskelett-Element und das zweite Exoskelett-Element mit ersten Aufnahmen, eingerichtet zur Aufnahme einer ersten Aktuator-Einheit zum steuerbaren Verschieben des zweiten Exoskelett-Elements entlang der gekrümmten Trajektorie relativ zum ersten Exoskelett-Element, versehen.

Dadurch kann eine Bewegung des Menschen, bspw. das Beugen und Strecken der Wirbelsäule bzw. des Oberkörpers, aktiv unterstützt werden.

Vorzugsweise sind das erste Exoskelett-Element und das zweite Exoskelett-Element mit zweiten Aufnahmen, eingerichtet zur Aufnahme einer zweiten Aktuator-Einheit zum steuerbaren Verschieben des zweiten Exoskelett-Elements entlang der gekrümmten Trajektorie relativ zum ersten Exoskelett-Element versehen, wobei die ersten Aufnahmen und die zweiten Aufnahmen zu einer durch die gekrümmte Trajektorie aufgespannten Ebene versetzt angeordnet sind.

Dadurch kann ein Drehmoment durch eine Achse rechtwinklig zur Führungsrichtung erzeugt werden, das dazu genutzt werden kann, ein seitliches Neigen oder ein Drehen um die Hochachse der Wirbelsäule aktiv zu unterstützen.

Vorzugsweise ist das erste Exoskelett-Element und/oder das zweite Exoskelett-Element mit einer Sensoreinheit zum Bestimmen eines Abstandes oder eines Winkels zwischen dem ersten Exoskelett-Element und dem zweiten Exoskelett-Element versehen.

Somit kann eine Steuerung der Bewegungsunterstützung noch präziser erfolgen.

Vorzugsweise umfasst ein Verfahren zur Reduktion von während einer Bewegung auf eine Wirbelsäule eines Menschen wirkenden Kräften ein aneinander Anreihen der Exoskelett-Elemente des Systems, ein Herstellen einer Anbindung des Systems an den Körper des Menschen, ein Bestimmen eines gewillkürten Streckens oder Beugens der Wirbelsäule des Menschen und in Reaktion auf das Bestimmen, ein Verschieben des zweiten Exoskelett-Elements entlang der gekrümmten Trajektorie relativ zum ersten Exoskelett-Element.

Dadurch kann ein gewillkürtes Strecken oder Beugen der Wirbelsäule des Menschen unterstützt und die Belastung der Wirbelsäule bzw. der Wirbelsäulenmuskulatur reduziert werden, indem bspw. die sonst auf den oberen Bereich der Wirbelsäule wirkende Kräfte von dem System aufgenommen und umgeleitet werden.

Vorzugsweise umfasst das Bestimmen des gewillkürten Streckens oder Beugens der Wirbelsäule des Menschen ein Auswerten von an dem Menschen angebrachten Sensoren einer Sensor-Einheit.

Dadurch kann ein gewillkürtes Strecken oder Beugen der Wirbelsäule schneller und präziser erkannt werden.

Vorzugsweise umfasst das Verfahren ferner ein Einstellen der gekrümmten Trajektorie auf einen Bewegungsablauf des Menschen beim Strecken oder Beugen der Wirbelsäule.

Durch das Einstellen, kann eine Bewegung der Exoskelett-Elemente noch präziser an die Bewegung einer Wirbelsäule angepasst werden.

Vorzugsweise umfasst das Einstellen ein Reduzieren von Abweichungen zwischen einer Momentanpolbewegung der Wirbel der Wirbelsäule des Menschen und einer Momentanpolbewegung entsprechender Exoskelett-Elemente.

Dadurch kann eine weitgehend dem natürlichen Bewegungsablauf folgende Unterstützung der Wirbelsäule und der Wirbelsäulenmuskulatur erreicht werden.

### Kurze Beschreibung der Zeichnungen

Die Erfindung wird nachfolgend in der detaillierten Beschreibung anhand von Ausführungsbeispielen erläutert, wobei auf Zeichnungen Bezug genommen wird, in denen:
- Abb. 1 ein Beispiel für eine Anordnung eines erfindungsgemäßen Systems an einem Körper eines Menschen in schematischer Körperseitenansicht;
- Abb. 2 ein Beispiel für eine Anordnung von Exoskelett-Elementen (Exo-
- Wirbelkörpern) eines erfindungsgemäßen Systems relativ zu einer menschlichen Wirbelsäule in schematischer Körperseitenansicht;
- Abb. 3 ein Beispiel für eine Ausführungsform eines Exo-Wirbelkörper in
- schräger Seitenansicht;
- Abb. 4 ein Beispiel für zwei aneinander angereihte Exo-Wirbelkörper
- gemäß Abb. 3 in schräger Seitenansicht;
- Abb. 5 ein Beispiel für zwei aneinander angereihte Exo-Wirbelkörper gemäß einer weiteren Ausführungsform in schräger Seitenansicht;
- Abb. 6 die aneinander angereihten Exo-Wirbelkörper der Abb. 5 in ausgefahrener Position in Seitenansicht; und
- Abb. 7 ein Verfahren zur Reduktion von während einer Bewegung auf eine Wirbelsäule eines Menschen wirkenden Kräften zeigt.

Dabei sind in den Zeichnungen gleiche und funktional ähnliche Elemente durch gleiche Bezugszeichen gekennzeichnet.

### Beschreibung der Ausführungsformen

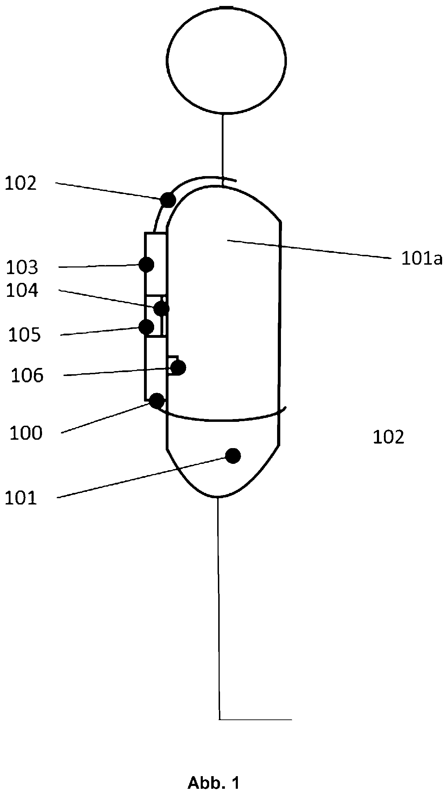

Abb. 1 zeigt ein anziehbares System 100, umfassend zwei Exo-Wirbelkörper 103, die parallel zur Wirbelsäule eines Menschen angeordnet sind. Das System 100 ist über Schnittstellen 102 an den Oberkörper 101a (bspw. an den Schultern) und an den Unterkörper 101b (bspw. am unteren Rücken/Becken) des Menschen angebunden. Die Schnittstellen 102 können, wie in Abb. 1 gezeigt, die Schultern oder das Becken (teilweise) umgreifen oder umspannen und/oder in (textile) Kleidungsstücke integriert sein, die eine Kraftübertragung zwischen dem System 100 und dem Körper des Benutzers ermöglichen. Ferner kann jeder Exo-Wirbelkörper 103 an dem Rücken anliegen, d. h. während der Verwendung mit dem Rücken des Benutzers (ggf. mittelbar über ein (textiles) Kleidungsstück, in welches das System 100 integriert ist) in Kontakt stehen.

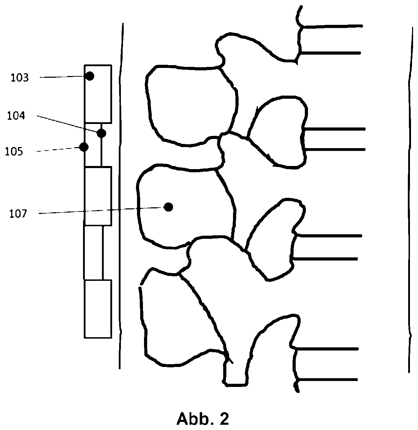

Wie in Abb. 1 und Abb. 2 gezeigt, kann ein Exo-Wirbelkörper 103 mehreren oder exakt einem menschlichen Wirbelkörper zugeordnet sein bzw. diese/diesen überspannen. Alternativ können auch mehrere Exo-Wirbelkörper 103 einem menschlichen Wirbelkörper zugeordnet sein bzw. diesen überspannen. Insbesondere kann ein Exo-Wirbelkörper 103 genau einem oder einer ganzzahligen Anzahl an menschlichen Wirbelkörpern zugeordnet sein bzw. diesen/diese überspannen. Ferner können, wie in Abb. 2 gezeigt, Exo-Wirbelkörper 103 und menschliche Wirbelkörper bei gestreckter Wirbelsäule horizontal nebeneinander angeordnet sein, wobei sich eine (gedachte) horizontale Linie durch einen Exo-Wirbelkörper 103 auch durch einen Schwerpunkt eines menschlichen Wirbelkörpers erstreckt.

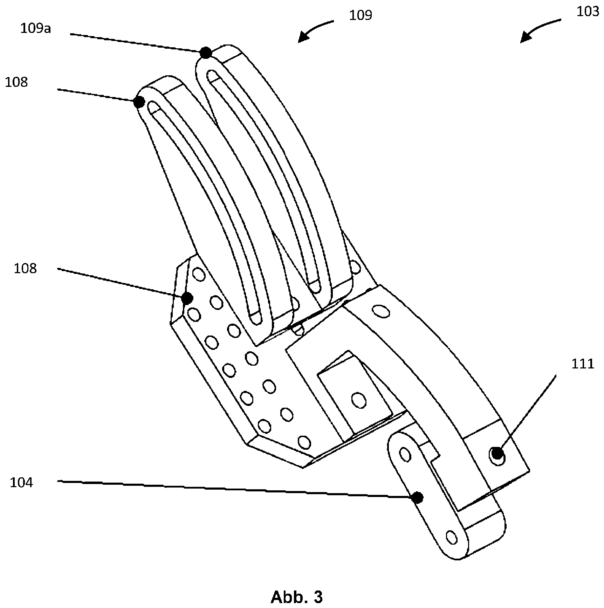

Wie in Abb. 1 und 2 gezeigt, sind ferner je zwei aufeinanderfolgende Exo-Wirbelkörper 103 über ein Führungselement 104 miteinander verbunden, wobei der unterste und der oberste Exo-Wirbelkörper 103 (d. h. die endständigen Exo-Wirbelkörper 103) auf der jeweils außenliegenden Seite über Schnittstellen 102 mit dem Nutzer 101 verbunden sind. Die Exo-Wirbelkörper 103 sind, wie weiter unten unter Bezugnahme auf Abb. 3 - Abb. 6 näher erläutert wird, so ausgestaltet, dass sie eine rotatorisch-lineare Relativbewegung ermöglichen. Dabei kann die Relativbewegung der Exo-Wirbelkörper 103 zueinander so ausgelegt sein, dass der Momentanpol ihrer Bewegung zumindest näherungsweise im Drehpunkt der korrespondierenden menschlichen Wirbelkörper liegt.

Zwischen zwei Exo-Wirbelkörpern 103 kann ferner jeweils eine Aktuator-Einheit 105 vorgesehen sein. Diese kann dazu eingesetzt werden, die Exo-Wirbelkörper 103 relativ zueinander (aktiv) zu verschieben und dadurch eine menschliche Bewegung zu unterstützen oder diese (ggf. richtungsabhängig) zu bremsen (bzw. zu stützen). Die Aktuator-Einheiten 105 können dazu bspw. mit Elektromotoren, künstlichen Muskeln, pneumatischen oder hydraulischen Aktuatoren, mechanischen Federn und Formgedächtnislegierungen versehen sein. Die Aktuator-Einheiten 105 können ferner mit einer Stromquelle, bspw. einem Akkumulator, oder einer Fluidquelle verbunden sein, die die Aktuator-Einheiten 105 mit elektrischer Energie bzw. einem (unter Druck stehenden) Fluid versorgt.

Das System 100 kann ferner eine Steuer-Einheit (nicht gezeigt) umfassen, die das System 100 und insbesondere die Aktuator-Einheiten 105 steuert und ggf. einen Betrieb des Systems 100 in verschiedenen Steuermodi ermöglicht. Ein Steuermodus kann bspw. vorsehen, dass die Aktuator-Einheiten 105 innerhalb eines bestimmten Bereichs in einen Freilauf betrieben werden, in dem menschliche Bewegungen frei möglich sind, oder nur gering unterstützt werden und Bewegungen außerhalb des bestimmten Bereichs verhindert, gebremst bzw. stärker unterstützt werden. Ein weiterer Steuermodus kann bspw. vorsehen, dass die Aktuator-Einheiten 105 jegliche Bewegungen des Menschen oder von dem Menschen manuell vorgegebene Bewegungen ("auf Knopfdruck") unterstützen. Ein weiterer Steuermodus kann bspw. Belastungsdaten auswerten, die z. B. durch Sensoren einer Sensor-Einheit 106 gemessen werden, wobei das Unterstützungsniveau auf Basis der Belastungsdaten angepasst wird. Bspw. können Elektromyografie- (EMG) oder Kraftsensoren genutzt werden, um das System 100 belastungsabhängig zu steuern. Beispielsweise kann das System 100 bei geringen Belastungen anfangs inaktiv sein und erst aktiv werden, wenn die Belastung einen kritischen Wert überschreitet.

Abb. 3 zeigt einen exemplarischen Exo-Wirbelkörper 103. Der in Abb. 3 gezeigte Exo-Wirbelkörper 103 umfasst ein Grundelement 108 (hier gezeigt in Form einer ebenen Platte), auf dem auf einer ersten (vertikalen) Verbindungsseite eine Führung 109 und auf einer zweiten (gegenüberliegenden) Verbindungsseite ein Führungselement 110 angeordnet ist. Die Führung 109 umfasst zwei parallele Führungsplatten 109a und 109b, die bogenförmige Aussparungen aufweisen. In Verwendung können die Exo-Wirbelkörper 103 relativ zur Wirbelsäule so angeordnet sein, dass der Mittelpunkt des Kreisbogens, auf dem sich zwei benachbarte Exo-Wirbelkörper 103 bewegen (d. h. der Momentanpol der Exo-Wirbelkörper 103) deckungsgleich mit dem rotatorischen Zentrum (bei einem als näherungsweise konstant angenommenen Momentanpol) der korrespondierenden, darunterliegenden menschlichen Wirbelkörper ist. Die exoskelettale Struktur erfährt durch dieses mechanische Verhalten bei einer Ventralflexion des Rückens des Benutzers somit eine Längung, die der Längung des Rückens des Benutzers entspricht, wodurch die Exo-Wirbelkörper 103 ortsfest am Rücken angeordnet werden können.

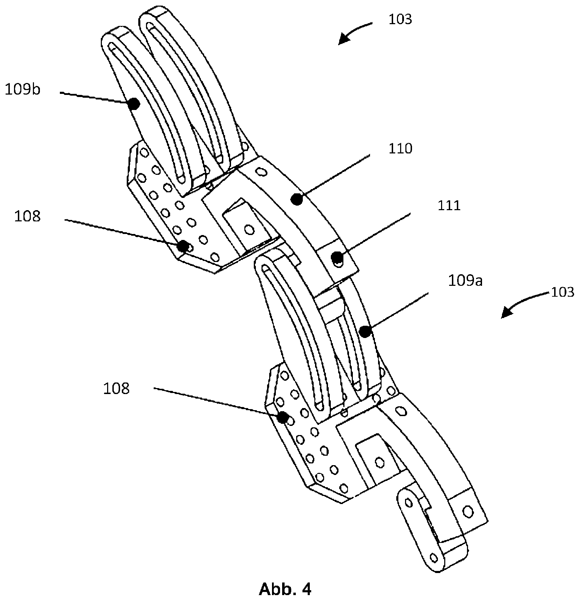

Das Führungselement 104, welches in Abb. 3 und Abb. 4 als Schlitten eines Kragarms 110 ausgebildet ist, umfasst zwei Aussparungen zur Aufnahme von Führungsstiften, welche, wie in Abb. 4 gezeigt, in den bogenförmigen Aussparungen gleitend, eine gleitende Lagerung des Führungselements 104 in der Führung 109 des angereihten Exo-Wirbelkörpers 103 realisieren. Das Führungselement 104 ist ferner über ein Drehlager 111 mit dem Grundelement 108 verbunden. Durch das Drehlager 111, welches in der Drehebene der ersten Anbindung verortet ist und eine (vorzugsweise auf den Momentanpol gerichtete) Drehachse besitzt, kann die Kette aus Exo-Wirbelkörpern 103 um einen rotatorischen Freiheitsgrad erweitert werden. Dieser weitere Freiheitsgrad ermöglicht neben dem Beugen und Strecken der durch die Exo-Wirbelkörper 103 gebildeten Exo-Wirbelsäule auch ein schräges Beugen und Strecken.

Das Grundelement 108 des Exo-Wirbelkörper 103 ist ferner mit Verbindungsmitteln in Form eines Lochrasters ausgestattet, welches eine Verbindung mit einer Schnittstelle 102 ermöglicht. An dem Lochraster können ferner die Aktuator-Einheiten 105 befestigt sein, bspw. an beiden Seiten des Kragarms 110. Ferner kann eine Aktuator-Einheit 105 an zwei aufeinander folgenden Exo-Wirbelkörpern 103 oder auch an mehr als zwei Exo-Wirbelkörpern 103 befestigt sein, die bspw. mittels eines Elektromotors und eines Seilzugs mit einem gleichen Übersetzungsverhältnis oder mit unterschiedlichen Übersetzungsverhältnissen gegeneinander verschoben werden. So kann bspw. eine Aktuator-Einheit 105 mittels eines Seilzugs, der sich zu den benachbarten Exo-Wirbelkörpern 103 erstreckt, diese in Richtung der Aktuator-Einheit 105 verschieben.

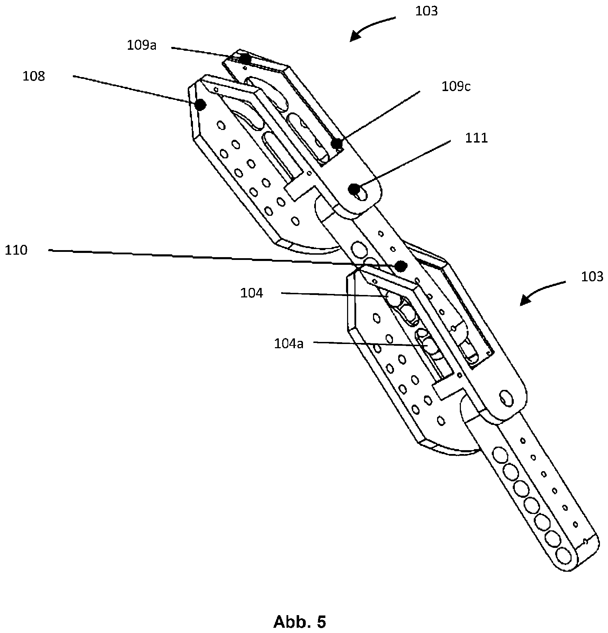

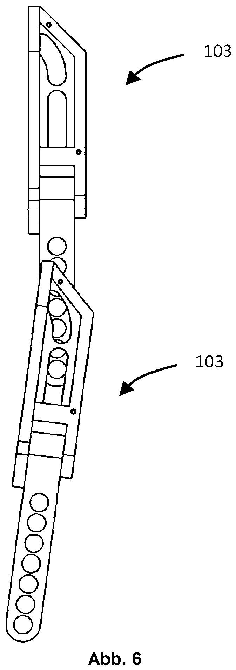

Abb. 5 und Abb. 6 zeigen ein weiteres Beispiel für zwei aneinander angereihte Exo-Wirbelkörper 103. Die in Abb. 5 und Abb. 6 gezeigten Exo-Wirbelkörper 103 unterscheiden sich von den in Abb. 3 und Abb. 4 gezeigten Exo-Wirbelkörpern 103 dadurch, dass anstatt einer bogenförmigen Aussparung jeweils zwei Aussparungen (Führungsbahnen) pro Platte 109a und 109b vorgesehen sind, wobei ein erstes Führungselement 104, welches durch einen ersten in eine Aussparung des Kragarms 110 einsteckbaren Stift gebildet wird, in der ersten Führungsbahn der ersten Platte 109a und ein in Führungsrichtung beabstandetes zweites Führungselement 104a, welches durch einen zweiten in eine Aussparung des Kragarms 110 einsteckbaren Stift gebildet wird, in einer zweiten Führungsbahn 109c der ersten Platte 109a gleitend gelagert ist. Das Vorsehen mehrerer Führungsbahnen und zugehöriger Führungsstifte erzeugt dabei ein Rotationsverhalten mit wanderndem Momentanpol. Um das Wanderverhalten des Momentanpols nachträglich anpassen zu können, sind die Führungsplatten 109a und 109b austauschbar gestaltet, so dass Platten mit anderen Führungsbahnverläufen eingesetzt werden können. Dadurch dass sich über die beschriebene Führungstechnik komplexe Momentanpolverläufe realisieren lassen, können auch eine größere Anzahl an Rückenwirbeln mit nur einem Exo-Wirbelkörper 103 überspannt werden.

Wie in Abb. 5 und Abb. 6 gezeigt, umfasst der Kragrarm 110 ferner eine Reihe an Aussparungen, so dass sowohl der Abstand der einsteckbaren Stifte zueinander als auch zum Schwerpunkt des jeweiligen Exo-Wirbelkörpers 103 einstellbar ist. Dabei versteht es sich, dass alternativ zu der Reihe an Aussparungen auch ein stufenloser Mechanismus zum Einstellen des Abstands der einsteckbaren Stifte zueinander als auch zum Schwerpunkt des jeweiligen Exo-Wirbelkörpers 103 realisiert sein kann, bspw. mittels einer durchgehenden Aussparung, in der die Stifte an beliebigen Positionen festgeklemmt werden können.

Die in Abb. 1 bis Abb. 6 gezeigten Systeme 100 dienen der Unterstützung der Wirbelsäule bzw. der Wirbelsäulenmuskulatur. Die durch die gezeigten Exo-Wirbelkörper 103 bildbare exoskelettale Struktur besteht dabei aus mehreren in Reihe geschalteten, sich wiederholenden Exo-Wirbelkörpern 103, die längs der Wirbelsäule angeordnet und gleitend miteinander verbunden sind. Durch entsprechende Auslegung oder Anpassung der Relativbewegungs-Trajektorien können die Exo-Wirbelkörper 103 zudem ortsfest auf dem Rücken angeordnet werden, da sie bei der Beugung oder Streckung des Rückens ihren Abstand zueinander verändern.

Da sich die Gesamtstruktur somit bei einer Beugung des Rückens nicht spannt, kann eine die natürliche Bewegungsfreiheit nicht oder allenfalls minimal einschränkende Unterstützung erreicht werden, bei der das System 100 keine mit Reibung verbundene Relativbewegung zum Körper ausführt. Ferner können die Exo-Wirbelkörper 103 so verbunden und gegeneinander geführt sein, dass sie bei einer Rückenbeugung einen relativen Drehpunkt zueinander besitzen, welcher mit dem effektiven Drehpunkt der darunterliegenden (menschlichen) Wirbelkörper deckungsgleich ist. Auf diese Weise ist die exoskelettale Struktur in der Lage, die Bewegung der menschlichen Wirbelsäule nachzuvollziehen, obwohl diese zur Biegelinie der Wirbelsäule versetzt angeordnet ist.

Durch Aktuator-Einheiten 105 zwischen den Exo-Wirbelkörpern 103 können des Weiteren Zug und/oder Druckkräfte und somit Drehmomente zwischen den Exo-Wirbelkörpern 103 erzeugt werden. Wenn die exoskelettale Struktur über eine geeignete Anbindung mit dem Körper des Benutzers verbunden ist, werden die in der exoskelettalen Struktur erzeugten Kräfte somit in den Oberkörper des Trägers übertragen und entlasten dadurch die Wirbelsäule und die Wirbelsäulenmuskulatur. Somit können Körperbewegungen, bei denen der Rücken gebeugt und gestreckt wird, unterstützt werden.

Das System 100 kann ferner durch einstellbar gestaltete Exo-Wirbelkörpern 103 (wie in Zusammenhang mit Abb. 5 und Abb. 6 beschrieben) an individuelle Körpereigenschaften (insb. Anthropometrie und Bewegungsmerkmale) des Benutzers angepasst werden. Insbesondere können durch einstellbar gestaltete Exo-Wirbelkörpern 103 Abstandsänderungen zwischen den Exo-Wirbelkörpern 103 in einen gewünschten Zusammenhang mit der Winkeländerung zwischen den Exo-Wirbelkörpern 103 gesetzt werden. Somit kann das System 100 individuell an die Anatomie des Benutzers angepasst werden, wodurch ein optimales Unterstützungsverhalten ermöglicht wird. Ferner ist das System 100 durch Hinzufügen oder Weglassen von Exo-Wirbelkörpern 103 oder durch die Verwendung von Exo-Wirbelkörpern 103 unterschiedlicher Abmessungen in der Länge skalierbar. Zudem lässt sich die Steifigkeit/Nachgiebigkeit/Elastizität der Exo-Wirbelkörper 103 über die geometrische Gestalt und die verwendeten Materialien einstellen.

Allgemein wird somit ein System 100 geringer Komplexität bereitgestellt, bei dem die geometrische Gestalt und die verwendeten Materialien zur Einstellung der Bewegungseigenschaften genutzt werden können und nur lineare Aktuierungsbewegungen benötigt werden. Zudem wird ein System 100 bereitgestellt, das eine hohe Anzahl an Gleichteilen aufweist. Das System 100 ermöglicht ferner die Realisierung einer definierten Beziehung relativer Rotation zu Translation während menschlicher Bewegungen durch vorgegebene Realativtrajektorien der Exo-Wirbelkörper 103 zueinander.

Das System 100 kann, wie oben ausgeführt, zur Unterstützung von Menschen eingesetzt werden, die zum einen ergonomisch ungünstige oder aber repetitive bzw. über einen längeren Zeitraum zu erfüllende Aufgaben ausführen müssen oder zum anderen Funktionseinbußen, z.B. aufgrund einer Verletzung am Rücken, erlitten haben. Darüber hinaus ist der Einsatz zur Stabilisierung von zumindest Teilen elastischer oder nachgiebiger technischer Elemente oder technischer Gelenke oder die Unterstützung anderer Lebewesen möglich. Dadurch kann zumindest ein Teil eines Körpers einer Person oder eines anderen Lebewesens oder auch eines technischen Systems (wie eines Industrieroboters) durch eine Kraftumleitung und -verstärkung entlastet bzw. geschont werden, was die Ausführung gewisser Tätigkeiten überhaupt erst ermöglicht.

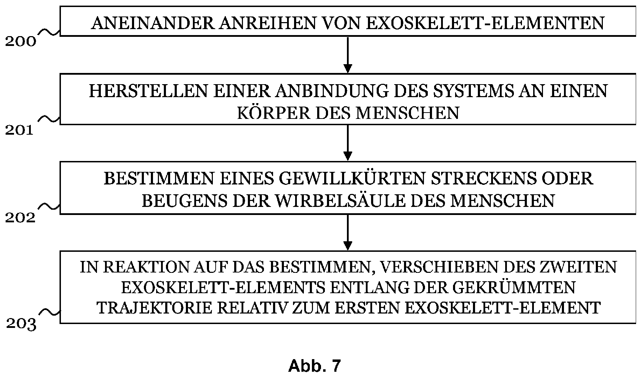

In diesem Zusammenhang zeigt Abb. 7 ein Verfahren zur Verwendung des Systems 100. Nach Analyse der kinematischen Gegebenheiten werden passende Exo-Wirbelkörper 103 aus einer Vielzahl unterschiedlicher Exo-Wirbelkörper 103 (insbesondere unterschiedlich hinsichtlich ihrer Abmessungen und der verwendeten Materialien) ausgesucht und bereitgestellt. Danach werden die Exo-Wirbelkörper 103, wie in Schritt 200, angegeben aneinander angereiht und in Schritt 201 an den Körper eines Menschen, eines anderen Lebewesens oder auch eines technischen Systems angebunden. Gegebenenfalls kann das System 100 bei Verwendung von einstellbaren Exo-Wirbelkörpern 103 (wie in Abb. 5 und Abb. 6 gezeigt) an einen Bewegungsablauf des Körpers wie oben beschrieben abgestimmt werden, indem bspw. Abweichungen zwischen einer Momentanpolbewegung der Wirbel der Wirbelsäule des Menschen und der Momentanpolbewegung der Exoskelett-Elemente 103 durch das Anpassen der Trajektorie reduziert werden. Wird mittels einer Sensor-Einheit 206 ein gewillkürtes Strecken oder Beugen der Wirbelsäule erkannt (Schritt 202) können in Schritt 203, in Reaktion auf das Bestimmen, die Aktuator-Einheiten 105 angesteuert werden, so dass sich die Exo-Wirbelkörper 103 gegeneinander Verschieben und in Folge dessen die durch das System 100 gebildete exoskelettale Struktur streckt oder beugt.

### Bezugszeichenliste

100 System
101a Oberkörper
101b Unterkörper
102 Schnittstelle
103 Exoskelett-Element
104, 104a Führungselement
105 Aktuator-Einheit
106 Sensor-Einheit
107 menschliche Wirbelkörper
108 Grundelement
109, 109a Führung
109b, 109c Führung
110 Kragarm
111 Drehlager

## Patentansprüche

1. System (100) zur Reduktion von auf eine Wirbelsäule eines Menschen wirkenden Kräften, aufweisend eine Vielzahl von aneinander anreihbaren Exoskelett-Elementen (103); wobei
zumindest zwei der aneinander anreihbaren Exoskelett-Elemente (103) eingerichtet sind, während der Verwendung des Systems (100) an den Körper des Menschen angebunden zu sein; und
ein erstes Exoskelett-Element (103) der aneinander anreihbaren Exoskelett-Elemente (103) eine Führung (109) umfasst, welche eingerichtet ist, ein zweites Exoskelett-Element (103) der aneinander anreihbaren Exoskelett-Elemente (103) entlang einer gekrümmten Trajektorie relativ zum ersten Exoskelett-Element (103) zu führen und beim Führen des zweiten Exoskelett-Elements (103) entlang der gekrümmten Trajektorie relativ zum ersten Exoskelett-Element (103) eine überlagerte translatorische und rotatorische Relativbewegung zwischen dem ersten Exoskelett-Element (103) und dem zweiten Exoskelett-Element (103) zu erzwingen;
**dadurch gekennzeichnet, dass**
das System (100) ferner eine Aktuator-Einheit (105) umfasst, wobei die Aktuator-Einheit (105) zum steuerbaren Verschieben des zweiten Exoskelett-Elements (103) relativ zum ersten Exoskelett-Element (103) eingerichtet ist.

2. System (100) nach Anspruch 1, wobei beim Führen des zweiten Exoskelett-Elements (103) entlang der gekrümmten Trajektorie relativ zum ersten Exoskelett-Element (103) die Gangpolbahn des zweiten Exoskelett-Elements (103) das erste und das zweite Exoskelett-Element (103) nicht schneidet.

3. System (100) nach einem der Ansprüche 1 bis 2, wobei beim Führen des zweiten Exoskelett-Elements (103) entlang der gekrümmten Trajektorie relativ zum ersten Exoskelett-Element (103) die Gangpolbahn des zweiten Exoskelett-Elements (103) relativ zum ersten und zweiten Exoskelett-Element (103) wirbelsäulenseitig versetzt liegt.

4. System (100) nach einem der Ansprüche 1 bis 3, wobei ein erstes Führungselement (104) des zweiten Exoskelett-Elements (103) in der Führung (109) gleitend lagerbar ist, wobei das zweite Exoskelett-Element (103) bei einer Verschiebung des ersten Führungselements (104) in der Führung (109) entlang der gekrümmten Trajektorie relativ zum ersten Exoskelett-Element (103) geführt wird.

5. System (100) nach einem der Ansprüche 1 bis 4, wobei das zweite Exoskelett-Element (103) ein Grundelement (108) aufweist und das erste Führungselement (104) des zweiten Exoskelett-Elements (103) relativ zum Grundelement (108) drehbar gelagert ist, wobei eine Drehung des ersten Führungselements (104) des zweiten Exoskelett-Elements (103) relativ zum Grundelement (108) es ermöglicht, das zweite Exoskelett-Element (103) entlang einer zweiten gekrümmten Trajektorie relativ zum ersten Exoskelett-Element (103) zu führen.

6. System (100) nach Anspruch 4 oder 5, wobei ein zweites Führungselement (104a) des zweiten Exoskelett-Elements (103) in der Führung (109) gleitend lagerbar ist, wobei das erste Führungselement (104) und das zweite Führungselement (104a) voneinander entlang einer Geraden beabstandet sind.

7. System (100) nach Anspruch 6, wobei die Gerade während der Verwendung des Systems (100) in einer durch einen Abschnitt der gekrümmten Trajektorie aufgespannten Ebene liegt.

8. System (100) nach Anspruch 7, wobei ein Abstand des ersten Führungselements (104) zum zweiten Führungselement (104a) oder zum Schwerpunkt des zweiten Exoskelett-Elements (103) stufenlos oder in Stufen einstellbar ist, wobei unterschiedliche Abstände unterschiedlich gekrümmte Trajektorien bewirken.

9. System (100) nach einem der Ansprüche 1 bis 8, wobei die Führung (109) stufenlos oder in Stufen einstellbar ist, wobei unterschiedliche Einstellungen unterschiedlich gekrümmte Trajektorien bewirken.

10. System (100) nach einem der Ansprüche 1 bis 9, wobei das erste Exoskelett-Element (103) und das zweite Exoskelett-Element (103) mit ersten Aufnahmen, eingerichtet zur Aufnahme einer ersten Aktuator-Einheit (105) zum steuerbaren Verschieben des zweiten Exoskelett-Elements (103) entlang der gekrümmten Trajektorie relativ zum ersten Exoskelett-Element (103), versehen sind.

11. System (100) nach Anspruch 10, wobei das erste Exoskelett-Element (103) und das zweite Exoskelett-Element (103) mit zweiten Aufnahmen, eingerichtet zur Aufnahme einer zweiten Aktuator-Einheit (105) zum steuerbaren Verschieben des zweiten Exoskelett-Elements (103) entlang der gekrümmten Trajektorie relativ zum ersten Exoskelett-Element (103) versehen sind, wobei die ersten Aufnahmen und die zweiten Aufnahmen zu einer durch die gekrümmte Trajektorie aufgespannten Ebene versetzt angeordnet sind.

12. System (100) nach Anspruch 11, wobei das erste Exoskelett-Element (103) und/oder das zweite Exoskelett-Element (103) mit einer Sensoreinheit zum Bestimmen eines Abstandes oder eines Winkels zwischen dem ersten Exoskelett-Element (103) und dem zweiten Exoskelett-Element (103) versehen ist.

13. Verfahren zur Reduktion von insbesondere auf eine Wirbelsäule eines Menschen wirkenden Kräften, umfassend:
aneinander Anreihen (200) von Exoskelett-Elementen (103) eines Systems (100) nach einem der Ansprüche 1 bis 12;
Herstellen (201) einer Anbindung des Systems (100) an einen Körper des Menschen;
Bestimmen (202) eines gewillkürten Streckens oder Beugens der Wirbelsäule des Menschen; und
in Reaktion auf das Bestimmen, Verschieben (203) des zweiten Exoskelett-Elements (103) entlang der gekrümmten Trajektorie relativ zum ersten Exoskelett-Element (103).

14. Verfahren nach Anspruch 13, wobei das Bestimmen (202) des gewillkürten Streckens oder Beugens der Wirbelsäule des Menschen ein Auswerten von an dem Menschen angebrachten Sensoren einer Sensor-Einheit (106) umfasst.

15. Verfahren nach Anspruch 13 oder 14, ferner umfassend ein Einstellen der gekrümmten Trajektorie auf einen Bewegungsablauf des Menschen beim Strecken oder Beugen der Wirbelsäule.

16. Verfahren nach Anspruch 15, wobei das Einstellen ein Reduzieren von Abweichungen zwischen einer Momentanpolbewegung der Wirbel der Wirbelsäule des Menschen und einer Momentanpolbewegung entsprechender Exoskelett-Elemente (103) umfasst.

## Claims

1. System (100) for reducing forces acting on a person's spine, said system having a plurality of joinable exoskeleton elements (103); wherein
at least two of the joinable exoskeleton elements (103) are designed to be connected, during use of the system (100), to the person's body; and
a first exoskeleton element (103) of the joinable exoskeleton elements (103) comprises a guide (109) which is designed to guide a second exoskeleton element (103) of the joinable exoskeleton elements (103) along a curved trajectory relative to the first exoskeleton element (103) and to enforce a superimposed translational and rotational relative movement between the first exoskeleton element (103) and the second exoskeleton element (103) when guiding the second exoskeleton element (103) along the curved trajectory relative to the first exoskeleton element (103);
**characterised in that**
the system (100) further comprises an actuator unit (105), wherein the actuator unit (105) is designed to controllably displace the second exoskeleton element (103) relative to the first exoskeleton element (103).

2. System (100) according to claim 1, wherein the moving centrode of the second exoskeleton element (103) does not intersect the first and second exoskeleton elements (103) during guiding of the second exoskeleton element (103) along the curved trajectory relative to the first exoskeleton element (103).

3. System (100) according to any of claims 1 to 2, wherein the moving centrode of the second exoskeleton element (103) is offset relative to the first and second exoskeleton elements (103) on the spine side during guiding of the second exoskeleton element (103) along the curved trajectory relative to the first exoskeleton element (103).

4. System (100) according to any of claims 1 to 3, wherein a first guide element (104) of the second exoskeleton element (103) can be mounted to slide in the guide (109), wherein the second exoskeleton element (103) is guided along the curved trajectory relative to the first exoskeleton element (103) during a displacement of the first guide element (104) in the guide (109).

5. System (100) according to any of claims 1 to 4, wherein the second exoskeleton element (103) has a base element (108) and the first guide element (104) of the second exoskeleton element (103) is rotatably mounted relative to the base element (108), wherein a rotation of the first guide element (104) of the second exoskeleton element (103) relative to the base element (108) enables the second exoskeleton element (103) to be guided relative to the first exoskeleton element (103) along a second curved trajectory.

6. System (100) according to claim 4 or 5, wherein a second guide element (104a) of the second exoskeleton element (103) can be mounted to slide in the guide (109), wherein the first guide element (104) and the second guide element (104a) are spaced apart from each other along a straight line.

7. System (100) according to claim 6, wherein, during use of the system (100), the straight line is in a plane defined by a portion of the curved trajectory.

8. System (100) according to claim 7, wherein a distance from the first guide element (104) to the second guide element (104a) or to the centre of gravity of the second exoskeleton element (103) is continuously adjustable or adjustable in steps, wherein different distances result in differently curved trajectories.

9. System (100) according to any of claims 1 to 8, wherein the guide (109) is continuously adjustable or adjustable in steps, wherein different adjustments result in differently curved trajectories.

10. System (100) according to any of claims 1 to 9, wherein the first exoskeleton element (103) and the second exoskeleton element (103) are provided with first receptacles designed to receive a first actuator unit (105) for controllably displacing the second exoskeleton element (103) along the curved trajectory relative to the first exoskeleton element (103).

11. System (100) according to claim 10, wherein the first exoskeleton element (103) and the second exoskeleton element (103) are provided with second receptacles designed to receive a second actuator unit (105) for controllably displacing the second exoskeleton element (103) along the curved trajectory relative to the first exoskeleton element (103), wherein the first receptacles and the second receptacles are arranged offset from a plane defined by the curved trajectory.

12. System (100) according to claim 11, wherein the first exoskeleton element (103) and/or the second exoskeleton element (103) are provided with a sensor unit for determining a distance or an angle between the first exoskeleton element (103) and the second exoskeleton element (103).

13. Method for reducing forces acting, in particular, on a person's spine, comprising:
joining (200) exoskeleton elements (103) of a system (100) according to any of claims 1 to 12;
establishing (201) a connection of the system (100) to the person's body;
determining (202) a deliberate extension or flexion of the person's spine; and
in response to the determining, displacing (203) the second exoskeleton element (103) along the curved trajectory relative to the first exoskeleton element (103).

14. Method according to claim 13, wherein the determining (202) of the deliberate extension or flexion of the person's spine comprises evaluating sensors of a sensor unit (106) attached to the person.

15. Method according to claim 13 or 14, further comprising adjusting the curved trajectory to a movement pattern of the person when extending or flexing the spine.

16. Method according to claim 15, wherein the adjusting comprises reducing deviations between a movement of the instant centre of rotation of the vertebrae of the person's spine and a movement of the instant centre of rotation of corresponding exoskeleton elements (103).

## Revendications

1. Système (100) pour réduire des forces s'exerçant sur une colonne vertébrale d'un être humain, présentant une pluralité d'éléments exosquelettiques (103) juxtaposables les uns aux autres ; dans lequel
au moins deux des éléments exosquelettiques (103) juxtaposables les uns aux autres sont conçus pour être fixés au corps de l'être humain durant l'utilisation du système (100) ; et
un premier élément exosquelettique (103) parmi les éléments exosquelettiques (103) juxtaposables les uns aux autres comprend un guide (109) qui est conçu pour guider un second élément exosquelettique (103) parmi les éléments exosquelettiques (103) juxtaposables les uns aux autres le long d'une trajectoire incurvée par rapport au premier élément exosquelettique (103) et pour forcer un mouvement relatif translationnel et rotatif superposé entre le premier élément exosquelettique (103) et le second élément exosquelettique (103) durant le guidage du second élément exosquelettique (103) le long de la trajectoire incurvée par rapport au premier élément exosquelettique (103) ; **caractérisé en ce que**
le système (100) comprend en outre une unité d'actionnement (105), dans lequel l'unité d'actionnement (105) est conçue pour déplacer de manière contrôlable le second élément exosquelettique (103) par rapport au premier élément exosquelettique (103).

2. Système (100) selon la revendication 1, dans lequel le centrode du second élément exosquelettique (103) ne coupe pas les premier et second éléments exosquelettiques (103) durant le guidage du second élément exosquelettique (103) le long de la trajectoire incurvée par rapport au premier élément exosquelettique (103).

3. Système (100) selon l'une quelconque des revendications 1 à 2, dans lequel le centrode du second élément exosquelettique (103) est décalé par rapport aux premier et second éléments exosquelettiques (103) vers la colonne vertébrale durant le guidage du second élément exosquelettique (103) le long de la trajectoire incurvée par rapport au premier élément exosquelettique (103).

4. Système (100) selon l'une quelconque des revendications 1 à 3, dans lequel un premier élément de guidage (104) du second élément exosquelettique (103) peut être placé de manière coulissante dans le guide (109), dans lequel le second élément exosquelettique (103) est guidé le long de la trajectoire incurvée par rapport au premier élément exosquelettique (103) durant un glissement du premier élément de guidage (104) dans le guide (109).

5. Système (100) selon l'une quelconque des revendications 1 à 4, dans lequel le second élément exosquelettique (103) présente un élément de base (108) et le premier élément de guidage (104) du second élément exosquelettique (103) est monté à rotation par rapport à l'élément de base (108), dans lequel une rotation du premier élément de guidage (104) du second élément exosquelettique (103) par rapport à l'élément de base (108) permet le guidage du second élément exosquelettique (103) par rapport au premier élément exosquelettique (103) le long d'une seconde trajectoire incurvée.

6. Système (100) selon la revendication 4 ou 5, dans lequel un second élément de guidage (104a) du second élément exosquelettique (103) peut être placé de manière coulissante dans le guide (109), dans lequel le premier élément de guidage (104) et le second élément de guidage (104a) sont espacés l'un de l'autre le long d'une droite.

7. Système (100) selon la revendication 6, dans lequel la droite se situe dans un plan défini par une partie de la trajectoire incurvée durant l'utilisation du système (100).

8. Système (100) selon la revendication 7, dans lequel une distance entre le premier élément de guidage (104) et le second élément de guidage (104a) ou le centre de gravité du second élément exosquelettique (103) est réglable de manière continue ou graduelle, dans lequel différentes distances entraînent différentes trajectoires incurvées.

9. Système (100) selon l'une quelconque des revendications 1 à 8, dans lequel le guide (109) peut être réglé de manière continue ou graduelle, dans lequel différents réglages entraînent différentes trajectoires incurvées.

10. Système (100) selon l'une quelconque des revendications 1 à 9, dans lequel le premier élément exosquelettique (103) et le second élément exosquelettique (103) sont fournis avec des premiers logements, lesquels sont conçus pour recevoir une première unité d'actionnement (105) pour déplacer de manière contrôlable le second élément exosquelettique (103) le long de la trajectoire incurvée par rapport au premier élément exosquelettique (103).

11. Système (100) selon la revendication 10, dans lequel le premier élément exosquelettique (103) et le second élément exosquelettique (103) sont fournis avec des seconds logements, lesquels sont adaptés pour recevoir une seconde unité d'actionnement (105) pour déplacer de manière contrôlable le second élément exosquelettique (103) le long de la trajectoire incurvée par rapport au premier élément exosquelettique (103), dans lequel les premiers logements et les seconds logements sont décalés d'un plan défini par la trajectoire incurvée.

12. Système (100) selon la revendication 11, dans lequel le premier élément exosquelettique (103) et/ou le second élément exosquelettique (103) sont munis d'une unité de détection pour déterminer une distance ou un angle entre le premier élément exosquelettique (103) et le second élément exosquelettique (103).

13. Procédé pour réduire des forces s'exerçant en particulier sur une colonne vertébrale d'un être humain, comprenant :
la juxtaposition les uns aux autres (200) d'éléments exosquelettiques (103) d'un système (100) selon l'une quelconque des revendications 1 à 12 ;
la fabrication (201) d'une fixation du système (100) dans un corps de l'être humain ;
la détermination (202) d'une extension ou d'une flexion délibérée de la colonne vertébrale de l'être humain ; et
en réponse à la détermination, le déplacement (203) du second élément exosquelettique (103) le long de la trajectoire incurvée par rapport au premier élément exosquelettique (103).

14. Procédé selon la revendication 13, dans lequel la détermination (202) de l'extension ou de la flexion délibérée de la colonne vertébrale de l'être humain comprend une évaluation de capteurs d'une unité de détection (106) montés sur l'être humain.

15. Procédé selon la revendication 13 ou 14, comprenant en outre un réglage de la trajectoire incurvée sur un profil de mouvement de l'être humain durant l'extension ou la flexion de la colonne vertébrale.

16. Procédé selon la revendication 15, dans lequel le réglage comprend une réduction de déviations entre un mouvement du centre instantané de rotation des vertèbres de la colonne vertébrale de l'être humain et un mouvement du centre instantané de rotation d'éléments exosquelettiques (103) correspondants.
